(19) Europäisches Patentamt
European Patent Office
Office européen des brevets

(11) EP 4 173 640 A1

(12) **EUROPEAN PATENT APPLICATION**
published in accordance with Art. 153(4) EPC

(43) Date of publication:
**03.05.2023 Bulletin 2023/18**

(21) Application number: **21833985.1**

(22) Date of filing: **30.06.2021**

(51) International Patent Classification (IPC):
***A61K 47/42*** (2017.01) ***A61K 35/17*** (2015.01)
***A61K 39/395*** (2006.01) ***A61P 31/00*** (2006.01)
***A61P 35/00*** (2006.01) ***C07K 16/18*** (2006.01)
***C07K 19/00*** (2006.01) ***C12N 5/0783*** (2010.01)

(52) Cooperative Patent Classification (CPC):
**A61K 35/17; A61K 39/395; A61K 47/42; A61P 31/00; A61P 35/00; C07K 16/18; C07K 19/00; C12N 5/06**

(86) International application number:
**PCT/JP2021/024808**

(87) International publication number:
**WO 2022/004805 (06.01.2022 Gazette 2022/01)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **30.06.2020 JP 2020113083**

(71) Applicant: **GAIA BioMedicine Inc.**
**Tokyo 162-0821 (JP)**

(72) Inventors:
- **HARADA, Yui**
  **Fukuoka 8190395 (JP)**
- **YONEMITSU, Yoshikazu**
  **Fukuoka 8190395 (JP)**

(74) Representative: **Vossius & Partner Patentanwälte Rechtsanwälte mbB**
**Siebertstraße 3**
**81675 München (DE)**

Remarks:
The complete document including Reference Table(s) and the Sequence Listing(s) can be downloaded from the EPO website

(54) **METHOD FOR STABILIZING BINDING OF ANTIBODY TO NK CELL AND USE THEREOF**

(57) The object of the invention is to stably bind an antibody to NK cells. A method for stabilizing binding of an antibody and an NK cell is provided, which method uses a substance having a region I that can bind to a surface protein of the NK cell, and a region II that can bind to the antibody. The substance may further contain a linker portion for linkage in addition to the region 1 and the region II.

EP 4 173 640 A1

[Fig.8]

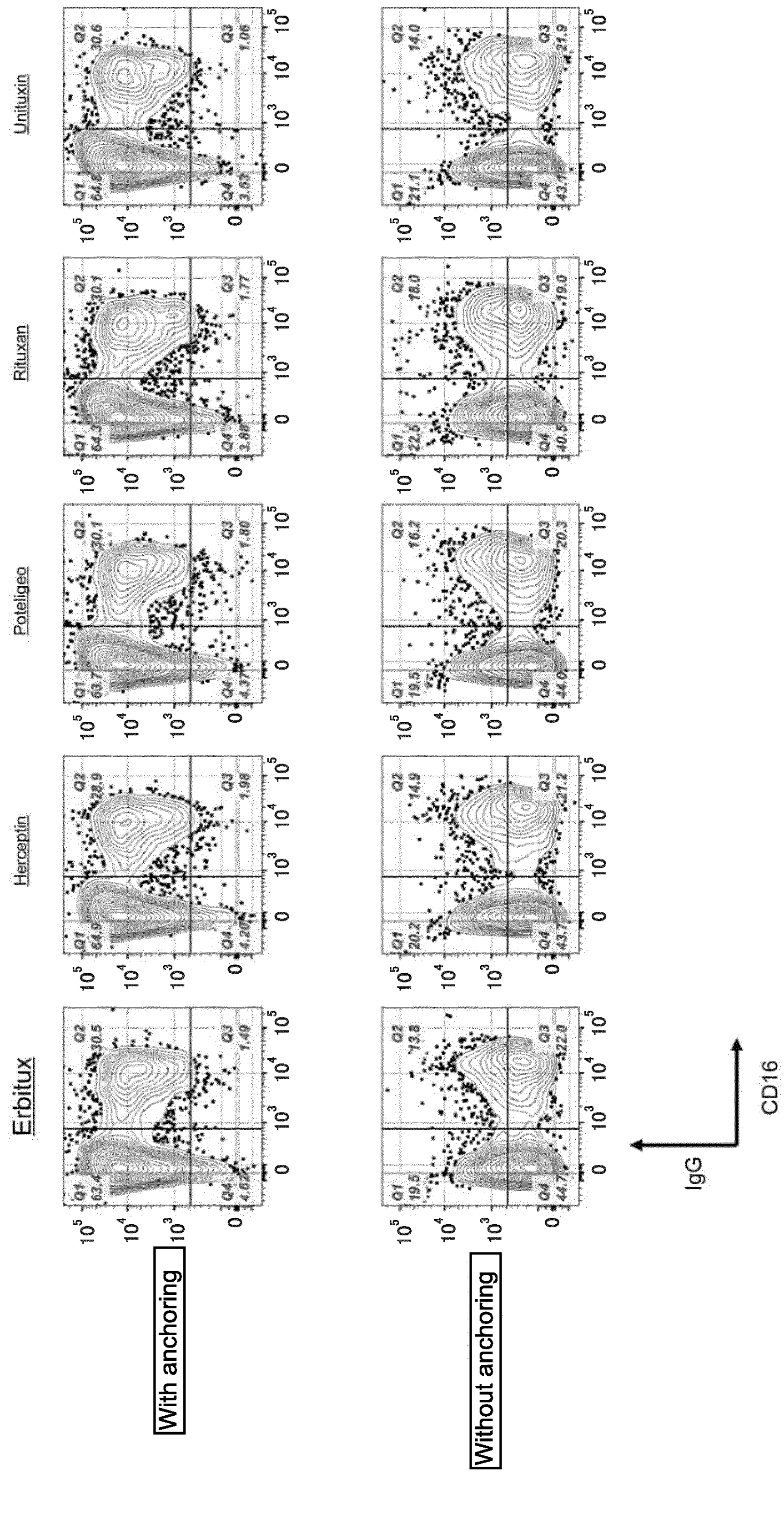

Erbitux
Herceptin
Poteligeo
Rituxan
Unituxin
With anchoring
Without anchoring
IgG
CD16

## Description

### Technical Field

The present invention relates to a method for stabilizing binding between NK cells and antibodies. The present invention is useful in the fields of immunotherapies of cancers using NK cells, manufacture of products for regenerative medicine and so forth for such therapies, and so forth.

### Background Art

Antibody drugs are designed to specifically bind to tumor cells that express a particular antigen on the cell surfaces, and thereby exert antibody-dependent cellular cytotoxicity (ADCC) activity to exhibit anti-tumor effects. However, it is known that they also bind to normal cells if the normal cells express the target antigen, and exhibit the ADCC activity as well (on-target off-tumor effect). In other words, the antibody drugs themselves cannot determine whether cells are tumor or not. In addition, this phenomenon is confirmed even in the absence of complement, if leukocytes are present, indicating that immune cells can also injure normal cells by the ADCC activity if antibodies bind to them. For example, administration of Rituxan, which targets CD20 expressed by B cells, is known to cause injury to normal B cells as a side effect thereof. To date, there is not known any method that can avoid this on-target off-tumor effect, and this is one of the biggest challenges facing the latest immune cell therapy, CAR-T cell therapy. It has been confirmed from various angles that it is mainly macrophage type cells that injure normal cells to which antibodies bind (Non-patent document 1).

In the mean time, natural killer cells (NK cells) are cytotoxic lymphocytes that act as a major factor in innate immunity and are important in the rejection of tumor cells and virus-infected cells. Immunotherapies of cancers using NK cells, in which NK cells amplified and activated in vitro are administered to patients, attract attention as a treatment method with relatively few side effects. The inventors of the present invention have developed novel NK-like cells with high cytotoxic activity and have also come up with the original idea of binding antibody drugs to such cells in vitro and then administering them to patients (Patent document 1).

Further, multispecific binding proteins that can bind to NK cell surface proteins have been proposed. For example, Patent document 2 proposes a chimeric fusion molecule having a tumor antigen binding domain and an immune cell binding domain for directing NK cells to antigen cells, wherein the antigen binding domain contains an isolated antibody or a fragment thereof of the antigen binding domain, the isolated antibody contains an immunoglobulin variable region and constant region, and the immune cell binding domain is a ligand specific for the NK cell receptor. In addition, Patent document 3 proposes use of a protein containing the NKp46 binding immunoglobulin region and a variable region thereof, such as antibodies and multispecific proteins, for specifically redirecting NK cells to lyse target cells. Furthermore, Patent document 4 proposes activation of NK cells by binding a multispecific binding protein that binds to the NKG2D receptor and CD16 to NK cells, and describes that the multispecific binding protein may contain an amino acid sequence at least 90% identical to the amino acids 234 to 332 of the human IgG1 antibody.

### Prior Art References

#### Patent documents

Patent document 1: Japanese Patent Unexamined Publication (Kokai) No. 2018-193303
Patent document 2: Japanese Patent Unexamined Publication (Kohyo) No. 2009-500346
Patent document 3: International Publication WO2017/114694 (Japanese Patent Unexamined Publication (Kohyo) No. 2019-503713)
Patent document 4: Japanese Patent Unexamined Publication (Kohyo) No. 2020-506971


#### Non-patent document

Non-patent document 1: Uchida J., et al., J. Exp. Med., 2004, Sugata K., et al., Sci. Rep., 2016

Summary of the Invention

Object to be achieved by the invention

While it is impossible for the macrophage type cells to distinguish between tumor cells and normal cells, NK cells can distinguish between tumor cells and normal cells. Therefore, if there can be created a circumstance in which NK cells are the sole effector of the ADCC activity provided by an antibody drug, the on-target off-tumor effect can be theoretically completely eliminated.

In order to create such a circumstance in which NK cells are the sole effector of the ADCC activity, it is desirable to avoid systemic administration of the antibody drug itself by intravenous infusion, or the like, and to administer the antibody drug bound to NK cells, considering the fact that it is not practical to eliminate macrophage-type cells from the whole body.

However, the investigations of the inventors of the present invention revealed that even if a target antibody drug is bound to NK cells, the target antibody drug would be removed from the NK cells in the presence of a certain component or in the presence of other competitive antibodies. Therefore, it is necessary to develop a technique that enables stable anchoring of antibodies on the NK cells.

Means for achieving the object

The inventors of the present invention have recently produced a fusion protein consisting of a region I that can bind to a surface protein of NK cells, a region II that can bind to an antibody, and a linker portion connecting the regions I and II, and have found that the binding between NK cells and antibodies can be stably maintained by this fusion protein. Thus, they accomplished the present invention.

The present invention provides the followings.

[1] A method for stabilizing binding of an antibody and an NK cell, which uses a substance having:

- a region I that can bind to a surface protein of the NK cell, and
- a region II that can bind to the antibody.

[2] The method according to 1, wherein the surface protein of NK cell is one selected from the group consisting of NKp46, NKp30, NKG2D, IL-15R, IL-2R, KIR3DS1, NKG2C, NKp80, NKp65, NKp44, LALRA1, LILRA2, DNAM-1, and 2B4.

[3] The method according to 1 or 2, wherein at least one of the region I and the region II is a single chain Fv fragment (scFv).

[4] The method according to any one of 1 to 3, wherein the antibody is an antibody drug for a treatment of a cancer or infectious disease.

[5] A method for producing a population of NK cells, which comprises the steps of:

(1) preparing a population of NK cells, an antibody, and a substance having a region I that can bind to a surface protein of the NK cells and a region II that can bind to the antibody; and
(2) adding the antibody to the population of NK cells in the presence of the substance to obtain a population of NK cells bound to the antibody, so that the NK cells are bound to the region I of the substance, the antibody is bound to the region II of the substance, and the substance stabilizes the binding between the antibody and the NK cells.

[6] The production method according to 5, wherein the antibody is an antibody drug for a treatment of a cancer or infectious disease.

[7] A pharmaceutical composition comprising a population of NK cells to which an antibody drug is bound, wherein binding of the antibody drug and the NK cells is stabilized by a substance having the following regions:

- a region I that can bind to a surface protein of the NK cells, and
- a region II that can bind to the antibody.

Effect of the invention

According to the present invention, the antibody drug should be substantially exclusively utilized by NK cells, and therefore it is expected that the side effect of injury of normal cells to which an antibody drug is bound by macrophage-type immune cells is suppressed.

Conventional methods for increasing binding activity of antibodies are based on modification of the sugar chain structure of the Fc region of antibodies (low fucose antibodies), modification of the amino acid sequence thereof, etc. However, the present invention uses a completely different approach to stably bind antibodies on NK cells. When antibodies are anchored to NK cells according to the present invention, a molecule that receives a favorable signal for the activation and survival of NK cells can be selected as the surface protein of the NK cells to which the antibodies are anchored.

Brief Description of the Drawings

[Fig. 1] Injury to Allo-WBC by NK cells in the case of using an antibody drug in combination.

[Fig. 2] Retention of antibody drug by NK cells in culture medium.

[Fig. 3] Influence of additives to culture medium on the binding of NK cells and antibody drug.

[Fig. 4] Influence of blood components on binding of NK cells and antibody drug.

[Fig. 5] Influence of competitive IgG on binding of NK cells and antibody drug.

[Fig. 6] Sequences used in the examples. The small letter portion is NKp46 sFv, the enclosed portion is protein G, and the underlined portion is a linker. In the nucleotide sequence, the positions (1) to (57) corresponds to the 30K signal peptide-coding region, (58) to (222) and (298) to (432) to the protein G-coding region, (433) to (477) to the (G4S)3 linker-coding region, (478) to (1212) to the scFv(NKp46)-coding region, (1213) to (1233) to the TEV protease-coding region, (1234) to (1251) and (1276) to (1293) to the His Tag-coding region, and (1252) to (1275) to the Strep Tag II-coding region.

[Fig. 7] Anchoring of antibody drug with prototype substance. (1) Highly active NK cells + PBS, (2) 350 μg/ml of a prototype substance is added to the highly active NK cells, reaction was allowed at room temperature for 1 hour, then 1 μg/ml of Herceptin was added, and reaction was allowed at room temperature for 1 hour, and (3) 350 μg/ml of a prototype substance was added to 1 μg/ml of Herceptin, reaction was allowed at room temperature for 1 hour, then the highly active NK cells were added, and reaction was allowed at room temperature for 1 hour.

[Fig. 8] Stabilization of cell-antibody binding by anchoring. Highly active NK cells were similarly suspended in PBS containing various antibody drugs in the presence or absence of a prototype substance, washed once with PBS, and subjected to antibody staining and measurement to analyze presence or absence of binding of each antibody drug. It was found that the binding between the cells and antibody was stabilized by anchoring with the prototype substance for all the antibody drugs.

Modes for Carrying out the Invention

The present invention relates to a method for stabilizing binding between an NK cell and an antibody using a substance having the following regions:

- a region I that can bind to a surface protein of the NK cell, and
- a region II that can bind to the antibody.

[Region I and region II]

(Region I that can bind to surface protein of NK cell)

As the NK cell surface protein to which the region I of the substance of the present invention binds, any of various protein molecules present on the surfaces of NK cells that do not inhibit the binding of antibody and CD16 (FcγRIII) on NK cells can be selected. Examples of such proteins include NKp46, NKp30, NKG2D, IL-15R, IL-2R, KIR3DS1, NKG2C, NKp80, NKp65, NKp44, LALRA1, LILRA2, DNAM-1, and 2B4.

Not to inhibit the binding of antibody and CD16 (FcγRIII) on NK cells means that, for example, when the substance binds to the cell surface protein and antibody, the substance has an appropriate three-dimensional structure (e.g., in height).

Among the NK cell surface proteins mentioned above, one that is expected to receive a favorable signal for activation or survival of NK cells upon binding is preferred as the NK cell surface protein to which the region I of the substance of the present invention binds. Examples of such a protein include NKp30, NKp46, NKG2D, and IL-15R. One particularly preferred example is NKp46, which has a larger number of ITAMs (immunoreceptor tyrosine-based activation motifs), and is considered to be an extremely important molecule among the activation type receptors of NK cells. Anti-NKp46 antibodies are commercially available as NK cell activators.

For the NK cell surface proteins mentioned above, molecules that bind to each of them are known.

NK cell surface protein: binding molecule (except for anti-X antibody)
NKp46: Cell Surface Vimentin (CSV)
NKp30: B7-H6
NKG2D: MICA, MICB, ULBP1 to 6
IL-15R: IL-15, IL-2
IL-2R: IL-15, IL-2
KIR3DS1: HLA-F, HLA-B
NKG2C: HLA-E
NKp80: AICL
NKp65: KACL
NKp44: MLL5, PCNA
LALRA1: HLA-B27 dimer
LILRA2: Soluble HLA
DNAM-1: CD112, CD155

Therefore, the region I of the substance of the present invention can be any one selected from the group consisting of CSV, B7-H6, MICA, MICB, ULBP1 to 6, IL-15, IL-2, IL-15, IL-2, HLA-F, HLA-B, HLA-E, AICL, KACL, MLL5, PCNA, HLA-B27 dimer, soluble HLA CD112, CD155, and CD48.

The region I may also be an antibody or antigen-binding fragment of antibody that can bind to any of the NK cell surface proteins described above. The antigen binding fragment of antibody may be single chain Fv fragment (scFv), dimeric scFv (di-scFv), diabody, triabody, tetrabody, Fab, F(ab')2, Fv F(ab')2, or Fv.

In one particularly preferred embodiment, the region I is any of the antigen binding domains of antibodies that bind to NKp46 described in Patent document 3 mentioned above, more specifically, the sequences of SEQ ID NOS: 119, 120, 121, and 122 mentioned in the sequence listing of that document, and the region I is preferably a polypeptide having the amino acid sequence of SEQ ID NO: 121 (having the amino acid sequence of the amino acids from the position 141 of the amino acid sequence of SEQ ID NO: 2 mentioned in the sequence listing of this description).

The region I may also be a peptide containing a region that can bind to an NK cell surface protein of any one of the binding molecules for NK cell surface proteins mentioned above, i.e., CSV, B7-H6, MICA, MICB, ULBP1 to 6, IL-15, IL-2, HLA-F, HLA-B, HLA-E, AICL, KACL, MLL5, PCNA, HLA-B27 dimer, soluble HLA, CD112, CD155, and CD48. In the sequence listing of this description, there are mentioned the region of B7-H6 that can bind to the NK cell surface protein as SEQ ID NO: 3, the region of MICA that can bind to the NK cell surface protein as SEQ ID NO: 4, the region of MICB that can bind to the NK cell surface protein as SEQ ID NO: 5, the region of IL-15 that can bind to the NK cell surface protein as SEQ ID NO: 6, the region of IL-2 that can bind to the NK cell surface protein as SEQ ID NO: 7, the region of HLA-F that can bind to the NK cell surface protein as SEQ ID NO: 8, the region of HLA-B that can bind to the NK cell surface protein as SEQ ID NO: 9, the region of HLA-E that can bind to the NK cell surface protein as SEQ ID NO: 10, the region of CD112 that can bind to the NK cell surface protein as SEQ ID NO: 11, the region of CD155 that can bind to the NK cell surface protein as SEQ ID NO:12, the region of CSV that can bind to the NK cell surface protein as SEQ ID NO: 32, the region of ULBP1 that can bind to the NK cell surface protein as SEQ ID NO: 33, the region of ULBP2 that can bind to the NK cell surface protein as SEQ ID NO: 34, the region of ULBP3 that can bind to the NK cell surface protein as SEQ ID NO: 35, the region of ULBP4 that can bind to the NK cell surface protein as SEQ ID NO:36, the region of ULBP5 that can bind to the NK cell surface protein as SEQ ID NO:37, the region of ULBP6 that can bind to the NK cell surface protein as SEQ ID NO:38, the region of AICL that can bind to the NK cell surface protein as SEQ ID NO:39, the region of KACL that can bind to the NK cell surface protein as SEQ ID NO:40, the region of PCNA that can bind to the NK cell surface protein as SEQ ID NO:41, and the region of CD48 that can bind to the NK cell surface protein as SEQ ID NO: 42. Polypeptides having a high sequence identity to any one of these amino acid sequences, and polypeptides that comprise an amino acid sequence derived from any one of those amino acid sequences by substitution, deletion, insertion, and/or addition of one or more amino acids and can bind to an NK cell surface protein can be used as well.

With regard to the present invention, unless especially mentioned, the number of amino acids substituted or the like in "an amino acid sequence derived by substitution, deletion, insertion, and/or addition of one or more amino acids" is not particularly limited for all the proteins, so long as the protein comprising the amino acid sequence has the desired function. However, the number of amino acids substituted or the like may be from 1 to 9 or from 1 to 4, or if substitutions are made to amino acids with similar properties, a larger number of substitutions may be included. With respect to the present invention, the term "identity" used for an amino acid sequence means percentage of the number of identical amino acids of two sequences determined by aligning the two sequences in an optimal manner, unless

especially stated. That is, identity can be calculated in accordance with the equation: Identity = (Number of matching positions/Total number of positions) × 100. Identity of amino acid sequences can be searched for and analyzed by using algorithms or programs well known to those skilled in the art (e.g., BLASTN, BLASTP, BLASTX, and ClustalW). When using programs, the parameters can be appropriately set by those skilled in the art, or the default parameters of each program may be used. High identity means that the identity is at least 80%, preferably 85% or higher, more preferably 90% or higher, further preferably 95% or higher, further preferably 97.5% or higher, further preferably 99% or higher, further preferably 99.8% or higher.

(Region II that can bind to antibody)

As the region II of the substance of the present invention, any of various protein molecules that can bind to an antibody and do not inhibit the binding of the antibody to CD16 (FcγRIII) on NK cells may be selected. Examples of such proteins include the Fc-binding ligands of IgG, of which examples include protein A (domain B (B1, B2, B3, B4, B5) of *Staphylococcus aureus*), protein G (domain B (B1, B2) of *Streptococcus* group C, domain C (C1, C2, C3) of *Streptococcus* group G), protein L, protein Z, protein LG, protein LA, protein AG, PAM, D-PAM, D-PAM-F, TWKTSRISIF, FGRLVSSIRY, Fc-III, Fc-III-(Sepharose), FcBP-2, Fc-III-4C, EPIHRSTLTALL, APAR, FcRM, HWRGWV, HYFKFD, HFRRHL, cyclo[(Nα-Ac)S(A)-, RWCitGWV, $D_2$AAG DAAG, RWHYFK-Lact-E], cyclo[(Nα-Ac)-Dap(A)-RWHYFK-Lact-E], cyclo[(Nα-Ac)-S(A)-RWHYFK-Lact-E], cyclo[Link-M-WFRHYK], NKFRGKYK, NARKFYKG, FYWHCLDE, FYCHWALE, FYCHTIDE, Dual 1/3, RRGW, KHRFNKD, Fc-III peptide (cyclic peptide with disulfide bonded thiol groups in cysteine residues, which has an ability to interact with the groove between the CH2 and CH3 domains in the Fc region of IgG, International Publication WO2001/045746; Science, 2000, Vol. 287, pp.1279-1283) or modifications thereof (Japanese Patent Unexamined Publication (Kokai) No. 2016-88906), YYWLHH, AV13 (GFRKYLHFRRHLL), and AV15 (VRLGWLLAPADLDAR) (US Patent No. 7,408,030), FcRM peptide (Chem. Bio. Chem., 2005, Vol. 6, pp.1242-1253), histidine (J. Chromatography, 1992, Vol. 604, pp.29-37), TG19318 (J. Molecular Recognition, 1998, Vol. 11, pp.128-133), TG19320 (J. Immunological Methods, 2002, Vol. 271, pp.77-88), IMG4K6R peptide (International Publication WO2013/027796; J. Biol. Chem., 2009, Vol. 284, pp.9986-9993), and IgA-binding peptides (International Publication WO2011/148952; J. Biol. Chem., 2012, Vol. 287, pp.43126-43136). For Fc-binding ligands for IgG, those skilled in the art can refer to Materials 2016, 9, 994; doi: 10.3390/ma9120994.

The region II may also be an antibody or an antigen-binding fragment of an antibody. The antigen binding fragment of an antibody may be single chain Fv fragment (scFv), dimeric scFv (di-scFv), diabody, triabody, tetrabody, Fab, $F(ab')_2$, or Fv.

The region II may be one that binds in an IgG Fc site-specific manner using a cross-linking agent. The cross-linking agent is a chemical substance used to covalently bond the IgG binding peptide and IgG Fc. The cross-linking agent used for the present invention can be appropriately selected by those skilled in the art, and can be a compound having at least two sites that can bind to a desired amino acid (e.g., lysine residue, cysteine residue, aspartic acid residue, glutamic acid residue, 2-aminosuberic acid, diaminopropionic acid, arginine, etc.). Examples thereof include, but are not limited to, cross-linking agents that preferably contain two or more succinimidyl groups such as DSG (disuccinimidyl glutarate), and DSS (disuccinimidyl suberate), cross-linking agents that preferably contain two or more imidic acid moieties such as DMA (dimethyl adipimidate dihydrochloride), DMP (dimethyl pimelimidate dihydrochloride), and DMS (dimethyl suberimidate dihydrochloride), and cross-linking agents having SS-bond such as DTBP (dimethyl 3,3'-dithiobispropionimidate dihydrochloride) and DSP (dithiobissuccinimidyl propionate). IgG-binding peptides modified with cross-linking agent can be added to IgG in a short time and with little side reaction.

In such a case as mentioned above, the region II has an amino acid residue that can react with the cross-linking agent in the sequence thereof. Examples of such an amino acid residue are those of protein-constituent amino acids such as lysine residue, cysteine residue, aspartic acid residue, and glutamic acid residue, and non-protein-constituent amino acids such as diaminopropionic acid and 2-aminosuberinic acid, and it is preferably lysine residue. The region II preferably has no or few (e.g., only one or two) other same amino acid residues as the amino acid residue that can react with the cross-linking agent in the sequence thereof. For example, if the amino acid residue that can react with the cross-linking agent is lysine residue, the peptide of the present invention preferably has no or few lysine residues at positions other than the position of amino acid residue to be reacted with the cross-linking agent in the sequence thereof.

Particularly preferred examples of such a peptide of the region II having an amino acid residue that can react with such a cross-linking agent are peptides having the sequences listed in the sequence listing of WO2016/186206 as SEQ ID NOS: 1 to 17, 36, and 37. They are listed in the sequence listing of this description as SEQ ID NOS: 13 to 29, 30 and 31, respectively. Polypeptides comprising a sequence having a high sequence identity to any one of these amino acid sequences, or an amino acid sequence derived from any one of these amino acid sequences by substitution, deletion, insertion, and/or addition of one or more amino acids, and can bind to an NK cell surface protein may be used as well.

Such peptides as mentioned above bind to the Fc domain of IgG. They also come close to a specific region of

IgG Fc, i.e., the residue Lys248 (corresponding to the 18th residue of human IgG CH2) or Lys246 (corresponding to the 16th residue of human IgG CH2) in human IgG Fc, preferably Lys248, indicated according to the Eu numbering.

For the reactions using such a cross-linking agent, WO2016/186206 mentioned above can be referred to. An example of this experimental scheme is shown below.

[Formula 1]

Osu: Succinimide, which competitively reacts against $H_2O$ and amine (hydrogencarbonate group + amino group).
$N_3$: Azide, which binds to alkyne through the Huisgen reaction.
○: Cyclic peptide Fc-III, Kd for IgG-Fc = 16 nM.
mal: Maleimide, which reacts with SH.

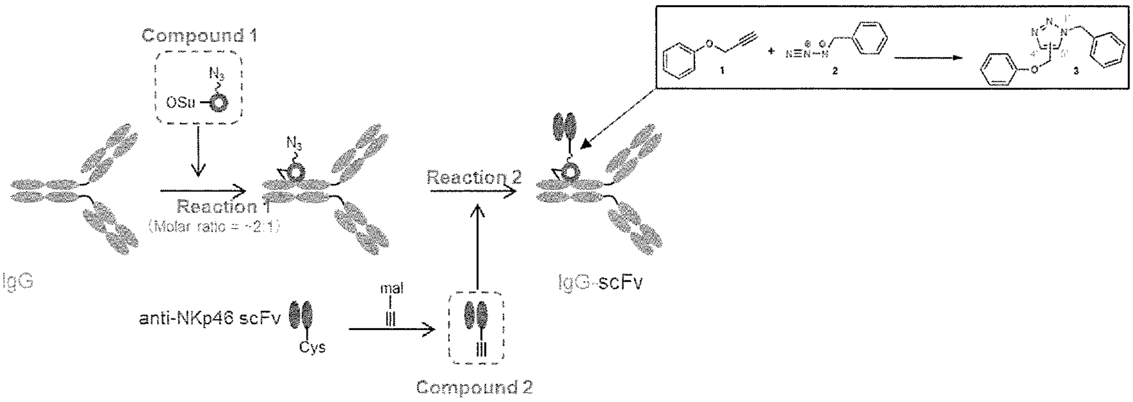


The region II may also be an aptamer or low molecular weight compound that recognizes the Fc region. The aptamer may be modified to such an extent that it can recognize the Fc region. Examples of the aptamer that recognizes the Fc region include human IgG aptamers (International Publication WO2007/004748; RNA, 2008, Vol. 14, pp.1154-1163), and so forth. Examples of the low molecular weight compound that recognizes the Fc region include mannosylerythritol lipid (J. Biomedical Materials Res. A, 2003, Vol. 65, pp. 379-385), transition metal ions (J. Appl. Polym. Sci., 89, 1567-1572; J. Chromatography B, 2003, Vol. 795, pp. 93-103), aromatic amines (J. Chromatography B, 2000, Vol. 740, pp.1-15), sulfamethazine (J. Chromatography B, 2003, Vol. 792, pp.177-185), and so forth.

The substance of the present invention may have a linker moiety connecting the region I and the region II. The linker moiety can have various structures so long as the region I can bind to a surface protein of NK cells, the region II can bind to an antibody, and the binding of the antibody to CD16 (FcγRIII) on the NK cells is not inhibited.

The linker may be, for example, a movable linker, which may be a peptide linker of 2 to 31 amino acids length. For example, the linker sequence may have about 16 amino acids length. Examples of preferred linker include peptides consisting of the sequence (G4S) × 3 or any one of SGGGGSGGGGSGGGGS (GS16), SG (GS2), SGGGGS (GS6), and SGGGGSGGGGSGGGGSGGGGSGGGGSGGGGS (GS31).

In one preferred embodiment, the linker is a rigid linker. For example, the rigid linker contains a sequence $(EAAAK)_n$, where n is 1 to 3. In one example, the rigid linker contains $(EAAAK)_n$, where n is 1 to 10 or about 1 to 100. For example, n is at least 1, at least 2, at least 3, at least 4, at least 5, at least 6, at least 7, at least 8, at least 9, or at least 10. In one example, n is less than 100. For example, n is less than 90, less than about 80, less than about 60, less than about 50, less than about 40, less than about 30, less than about 20, or less than about 10.

In one preferred embodiment, the linker is a cleavable linker. For example, the linker can be cleaved by a protease or peptidase.

(Preferred embodiments)

The amino acid sequence and the sequence of the polynucleotide encoding the amino acid sequence according to one of the particularly preferred embodiments where the substance of the present invention is a fusion protein are shown in Fig. 6 and the sequence listing.

(NK cells)

In general, NK cells are large granular lymphocytes that do not express the T cell receptor (TCR), the universal T cell marker, CD3, and the membrane immunoglobulin, B cell receptor, and are usually CD16-positive and CD56-positive in humans. NK cells can be easily distinguished by those skilled in the art on the basis of cell surface marker expression pattern, or the like. NK cells have cytotoxic activity, and the presence or absence and degree of cytotoxic activity can be measured by various known methods.

When referring to NK cells in the present invention, they includes the general NK cells mentioned above, as well as peripheral blood NK cells, cord blood NK cells, primary NK cells, cultured NK cells, highly active NK cells, and NK cells or NK-like cells of [1], [2], [3] and [4] mentioned below, unless otherwise stated.

[1] NK cells showing the following characteristics (1) and (2):

(1) CD16 positivity, CD56 high expression, and CD57 negativity.

(2) NKG2C positivity, NKG2A negativity to low expression, and CD94 positivity.

The highly active NK cells of [1] may show high CD16 expression. The highly active NK cells of [1] may also have the following characteristics, regardless of whether they highly express CD 16 or not:

(3) cytotoxic activity of 50% or higher as measured in co-culture of the NK cells as effector cells (E) and the K562 cells as target cells (T) at a mixing ratio (E:T) of 1:1.

The highly active NK cells of [1] can also be expressed as follows:

NK cells obtained by eliminating CD3-positive cells from peripheral blood mononuclear cells derived from a healthy human using CD3 beads (e.g., CliniMACS CD3, Miltenyi Biotech, Catalog No. 130-017-601), LD column (e.g., Miltenyi Biotech, Catalog No. 130-042-901), and a separation buffer (e.g., PBS containing 0.5% human AB serum (inactivated), and 2 mM EDTA), and culturing the obtained cell population for 14 days in an appropriate medium (e.g., Cosmedium 008 supplemented with 5% human AB serum (inactivated)), and showing the following characteristics (1) and (3):

(1) CD16 positivity, CD56 high expression, and CD57 negativity, and

(3) Cytotoxic activity of 50% or higher as measured in co-culture of the NK cells as effector cells (E) and the K562 cells as target cells (T) at a mixing ratio (E:T) of 1:1.

For details of the characteristics, and more specific production method of the highly active NK cells of [1], Japanese Patent Unexamined Publication (Kokai) No. 2018-193303 can be referred to.

[2] The following cells:

CCR5-positive, CCR6-positive, CXCR3-positive and CD3-negative cells.

The cells of [2] may also show high CD11c expression.

The cells of [2] can also be expressed as follows:

CCR5-positive, CCR6-positive, CXCR3-positive, integrin $\alpha$1-positive, integrin $\alpha$3-positive, integrin $\beta$3-negative, and CD3-negative cells, or CCR5-positive, CCR6-positive, CXCR3-positive, highly CD11a-expressing, highly CD11c-expressing, and CD3-negative cells, of which high expressions are determined by comparison with the expressions in a population of NK cells obtained from peripheral blood and not substantially cultured.

According to the studies of the inventors of the present invention, the cells of [2] show extremely high cytotoxic activity against solid tumors forming tumor masses. For details of the characteristics, and more specific production method of the highly active NK cells of [2], Japanese Patent Unexamined Publication (Kokai) No. 2019-170176 can be referred to.

[3] Highly active NK cells obtainable by the following method:

To mononuclear cells obtained from fresh peripheral blood or frozen apheresis blood, add CD3 beads (e.g., CliniMACS CD3, Miltenyi Biotech, 130-017-601 (5 $\mu$L per $1 \times 10^7$ cells)), further add CD34 beads (e.g., CliniMACS CD34, Miltenyi Biotech, 130-017-501 (2.5 $\mu$L per $1 \times 10^7$ cells)) in the case of using mononuclear cells obtained from frozen apheresis blood, suspend them, incubate the suspension at 4°C for 15 minutes, then add a separation buffer (e.g., PBS containing 0.5% human type AB serum (inactivated at 56°C for 30 minutes), and 2 mM EDTA), suspend them well, and centrifuge the suspension. Remove the supernatant, and suspend the cells in 0.5 mL of the separation buffer in such a number that the cell number in one LD column (e.g., Miltenyi Biotech, 130-042-901) should be $1 \times 10^8$ cells at the maximum. After adding 2 mL of the separation buffer to the LD column beforehand, add the cell suspension to

the LD column, and collect eluate from the LD column. Add additional 1 mL of the separation buffer to the LD column, and collect eluate. After centrifuging the collected eluates and removing the supernatant, suspend the cells in an appropriate medium (e.g., KBM501 medium containing either 5% human AB serum (inactivated at 56°C for 30 minutes) or 5% UltraGRO (AventaCell, HPCPLCRL10) supplemented with 2 U/mL of sodium heparin) at a density of $5 \times 10^5$ cells/mL in the case of using peripheral blood, or $1 \times 10^6$ cells/mL in the case of using frozen apheresis blood, and culture them up to day 14, with appropriately changing the medium.

For the specific production method of the highly active NK cells of [3], the description of Japanese Patent Application No. 2020-35297 can be referred to.

[4] Cells obtained by obtaining any of the cells of [1] to [3] with adding any one selected from the group consisting of IL-12, IL-15, and IL-18 at such a concentration that the purpose of the present invention can be achieved together with or instead of IL-2 at the time of culture. For the specific production method of such cells, Leong JW et al., Biol. Blood Marrow Transplant, 20 (2014), 463-473 can be referred to.

In this description, the present invention may be explained with reference to the case of using highly active NK cells as an example, but those skilled in the art can also understand other cases using cells subjected to an in vitro activation operation using some cytokine in accordance with the explanations.

(Cytotoxic activity)

For the present invention, the term activity or cytotoxic activity used for highly active NK cells, and so forth refers to an ability of subject cells (effector cells, E) to lyse target cells (T), unless especially stated. Cytotoxic activity can be expressed as the percentage (%) of target cells killed by effector cells, and is calculated in accordance with the following equation.

(Cell death observed in co-culture with effector cells - Spontaneous cell death (negative control))/(Maximum cell death (positive control) - Spontaneous cell death (negative control)) x 100

When cytotoxic activity is measured, in general, the mixing ratio of the effector cells to the target cells (E:T) and the time of co-culture of the effector cells and target cells can be appropriately determined according to the types and intensity of activity of cells to be used. When NK cells are used as the effector cells, the target cells may be, but are not limited to, K562 cells, acute myeloid leukemia cells, or chronic myeloid leukemia cells. The effector cells and target cells, and live cells and dead cells can be distinguished and quantified by using reagents such as antibodies labeled with a radioactive substance, fluorescent dye, or the like. When NK cells are used as the effector cells, the cytotoxic activity thereof can be measured by using the K562 cells as the target cells with the conditions of, for example, E:T of 1:0.05 to 10, preferably 1:0.1 to 5, more preferably 1:1, and an incubation time of 0.5 to 18 hours, preferably 1 to 12 hours.

For the present invention, the expression that the activity of NK cells or the like is high means that the cytotoxic activity is 50% or higher as measured by using the K562 cells as the target cells, mixing the cells at E:T of 1:1 and incubating them for 0.5 to 3 hours, more specifically 2 hours, unless especially stated. The activity should be preferably 60% or higher, more preferably 70% or higher.

For the present invention, the expression that certain cells (e.g., cells other than antigenic tumor cells, normal cells) are not injured by NK cells, or the like means that the cytotoxic activity is less than 5% as measured by mixing the cells with target cells (T) at E:T of 1:1, and incubating them for 0.5 to 3 hours, more specifically 2 hours, unless especially stated. The activity against the target cells is preferably less than 3%, more preferably less than 1% or undetectable.

[Method for producing population of NK cell]

The present invention also provides a method for producing a population of NK cells, which comprises the steps of:

(1) preparing a population of NK cells, an antibody, and a substance having a region I that can bind to a surface protein of the NK cells and a region II that can bind to the antibody; and
(2) adding the antibody to the population of NK cells in the presence of the substance to obtain a population of NK cells bound to the antibody, so that the NK cells are bound to the region I of the substance, the antibody is bound to the region II of the substance, and the substance stabilizes the binding between the antibody and the NK cells.

(Step (1): Preparation of population of NK cells or the like)

Raw material of the population of NK cell may be peripheral blood, cord blood, bone marrow and/or lymph nodes, or blood collected by apheresis method (apheresis blood). The raw material may also be one prepared from at least one kind of cells selected from hematopoietic stem cells derived from any stem cells selected from the group consisting of embryonic stem cells, adult stem cells, and induced pluripotent stem (iPS) cells, hematopoietic stem cells derived from cord blood, hematopoietic stem cells derived from peripheral blood, hematopoietic stem cells derived from bone marrow blood, cord blood mononuclear cells, and peripheral blood mononuclear cells. The donor of the raw material may be a patient himself/herself who will receive an immunotherapy using highly active NK cells or the like, a close relative of the patient, or a healthy person genetically unrelated to the patient. The donor may consist of a plurality of donors.

(Culture medium)

Examples of the culture medium used for culturing and activating cells as the raw material in order to obtain a population of NK cells include the KBM501 medium (Kojin Bio, containing 1,750 JRU/mL of IL-2), Cosmedium 008 (Cosmo Bio, containing 1,750 JRU/mL of IL-2), FKCM101 (Fukoku, containing no IL-2 or 175 IU/mL of IL-2), CellGro SCGM medium (CellGenix, Iwai Chemical), X-VIVO15 medium (Lonza, Takara Bio), Gibco (registered trademark) CTS (registered trademark) AIM V (registered trademark) Medium (Thermo Fisher Scientific, serum-free medium of known composition for growth and manipulation of T cells and dendritic cells), CTS OpTmizer T Cell Expansion Basal Medium (Thermo Fisher Scientific, for growth and proliferation of human T lymphocytes), IMDM, MEM, DMEM, RPMI 1640, and so forth, but not limited to these. Preferred examples are the KBM501 medium, FKCM101, and Cosmedium 008. For the present invention, the expression that culture of cells (to culture cells) means to maintain cells in a medium or a similar solution for a certain period of time for any purpose selected from the group consisting of maintaining cell viability, amplifying cells, and activating cells, unless especially stated. To carry out a treatment at a specific temperature for a certain period of time may be sometimes referred to as incubation (to incubate).

IL-2 may be added to the medium at such a concentration that the purpose of the invention can be achieved. The concentration of IL-2 may range from 2500 to 2813 IU/mL. IL-2 should preferably have a human amino acid sequence thereof, and be produced by a recombinant DNA technique for safety reasons. IL-2 concentration may be expressed in the national standard unit (Japan Reference Unit, JRU) and international unit (IU). 1 IU is approximately 0.622 JRU, and therefore 1,750 JRU/mL in the existing media is equivalent to approximately 2813 IU/mL.

Together with or instead of IL-2 described above, one selected from the group consisting of IL-12, IL-15, and IL-18 may be added at such a concentration that the purpose of the present invention can be achieved (Leong JW et al., Biol. Blood Marrow Transplant, 20 (2014) 463-473 mentioned above). The concentration of each may be 1 pg/mL to 1 μg/mL irrespective of the presence or absence or concentration of other cytokines. IL-2 should preferably have a human amino acid sequence thereof, and be produced by a recombinant DNA technique for safety reasons.

The medium may be supplemented with the subject's autologous serum, human type AB serum available from BioWhittaker and others, or donated blood human serum albumin available from the Japanese Red Cross Society. Autologous serum and human AB serum are preferably added at a concentration of 1 to 10%, and donated blood human serum albumin is preferably added at a concentration of 1 to 10%. Human platelet lysate (HPL) may be added together with or instead of serum. HPL is commercially available, and those of the UltraGRO™ series (AventaCell BioMedical), and so forth are commercially available. Sodium heparin may be further added to the medium, when HPL is used.

The medium may contain appropriate proteins, cytokines, antibodies, compounds, and other components, on condition that they do not impair the effectiveness of the NK cell culture. Cytokines may be IL-2, IL-12, IL-15, and IL-18 described above, as well as IL-3, IL-7, IL-21, stem cell factor (SCF), and/or FMS-like tyrosine kinase 3 ligand (Flt3L). All of these preferably have human amino acid sequences thereof, and are produced by a recombinant DNA technique for safety reasons.

The medium is preferably a serum-free medium. The serum-free medium preferably contains serum albumin, transferrin, and insulin. Serum-free media for culturing lymphocytes have been developed, and are commercially available, and they can be used for the present invention. One preferred example of serum-free medium is a basic medium supplemented with CTS Immune Cell SR (Thermo Fisher Scientific), which is commercially available as a composition that supports the proliferation of human T cells.

The medium may be replaced or replenished at any time after the start of culture, on condition that the desired culture effect is obtained, but the medium is preferably replaced or replenished every 3 to 5 days.

Culture vessels used for the culture include, but are not limited to, commercially available dishes, flasks, plates, and multi-well plates. Culture conditions are not particularly limited, so long as the culture effect of NK cells is not impaired, but culture conditions of 37°C, 5% $CO_2$, and saturated water vapor atmosphere are generally used. Culture period is not particularly limited, on condition that the desired culture effect is obtained.

(Antibody and antibody drug)

The term "antibody" used for the present invention refers to immunoglobulin of any class (isotype), unless otherwise stated. In the case of humans, there are five classes of antibodies: IgG, IgM, IgA, IgD, and IgE. When referring to antibody in the present invention, unless otherwise stated, it may be not only an antibody, but also an antibody drug having an antibody-derived sequence. When referring to antibody drug in the present invention, the species from which the antibody drug is derived is not limited, unless especially stated. The antibody drug used in the present invention may consist of mouse antibodies, chimeric antibodies, humanized antibodies, human antibodies, or antibodies without a sub-stem indicating their origin. In this description and the drawings, the present invention may be explained with reference to the case where the antibody is IgG as an example, but those skilled in the art can also understand other cases using antibodies of other classes by appropriately applying the explanations.

(IgG)

For the present invention, IgG includes IgG as well as molecules derived from IgG, unless especially stated. Molecules derived from IgG include antibody fragments. Examples thereof include Fab, scFv, $V_H$-$V_L$, scFv-$C_H$3, VhH, Dab, multimers thereof, and fusions thereof (Nature Biotechnology, Volume 23, pages 1126-1136 (2005)). Molecules derived from IgG also include single domain antibodies. Examples thereof include VHH, VNAR, and sdAb. Single domain antibodies can selectively bind to specific antigens and have a lower molecular weight than IgG and antibody fragments such as Fab and scFv. The VHH antibody (variable domain of heavy chain of heavy chain antibody) is a variable domain of antibody consisting only of H chain of camelids (llama, alpaca, etc.) (heavy chain antibody), which is highly stable, can be produced at low cost, and is expected to be used as an antibody drug (Front Immunol., 2017 Jun., 9;8:653).

For the present invention, IgG also includes antibody drugs having an IgG-derived sequence. Examples of antibody drugs having an IgG or IgG-derived sequence include mouse antibodies such as muromonab-CD3, ibritumomab tiuxetan, iodine 131 tositumomab, catumaxomab, blinatumomab, and moxetumomab pasudotox, chimeric antibodies such as abciximab, rituximab, basiliximab, infliximab, cetuximab, brentuximab vedotin, siltuximab, dinutuximab, and obiltoxaximab, humanized antibodies such as daclizumab, palivizumab, trastuzumab, gemtuzumab ozogamicin, alemtuzumab, omalizumab, efalizumab, bevacizumab, natalizumab, tocilizumab, ranibizumab, eculizumab, certolizumab pegol, mogamulizumab, pertuzumab, trastuzumab emtansine, obinutuzumab, vedolizumab, pembrolizumab, idarucizumab, mepolizumab, elotuzumab, ixekizumab, reslizumab, atezolizumab, ocrelizumab, inotuzumab ozogamicin, emicizumab, benralizumab, galcanezumab, fremanezumab, tildrakizumab, caplacizumab, ibalizumab, ravulizumab, romosozumab, and risankizumab, human antibodies such as adalimumab, panitumumab, golimumab, ustekinumab, canakinumab, ofatumumab, denosumab, ipilimumab, belimumab, raxibacumab, ramucirumab, nivolumab, secukinumab, evolocumab, alirocumab, necitumumab, daratumumab, brodalumab, olaratumab, bezlotoxumab, avelumab, durvalumab, dupilumab, bezlotoxumab, guselkumab, sarilumab, burosumab, erenumab, and lanadelumab, and antibodies not having sub-stem indicating the origin thereof such as emapalumab.

The antibody drug used in the present invention is preferably an antibody drug for a treatment of a cancer or infectious disease.

(Step (2): Stabilization of binding of antibody and NK cell)

The method of the present invention also comprises the step of adding the antibody to the population of NK cells in the presence of the substance of the present invention to obtain a population of NK cells bound to the antibody, so that the NK cells and the region I of the substance are bound, the antibody and the region II of the substance are bound, and the substance stabilizes the binding between the antibody and the NK cells. The NK cell population, the antibody, and the substance can be mixed immediately before use. For example, the NK cell population, antibody and substance can be maintained in separate containers, and mixed just before to several hours before administration to the subject.

When they are mixed, the order of addition can be arbitrarily determined. For example, after mixing and incubating the NK cell population and the substance, the antibody may be added, and the mixture may be further incubated, or after mixing and incubating the antibody and the substance, the NK cell population may be added, and the mixture may be further incubated.

The method for producing a population of NK cells may also comprises the step of removing antibodies that are not bound to NK cells. That is, the population of NK cells obtained by this production method contains NK cells and antibodies, and it is preferred that all the antibodies bind to the NK cells, and the NK cells are substantially free of antibodies that are not bound to the NK cells.

[Pharmaceutical composition]

The present invention also provides a pharmaceutical composition comprising a population of NK cells to which an antibody drug is bound, wherein binding of the antibody drug and the NK cells is stabilized by a substance having the following regions:

- a region I that can bind to a surface protein of the NK cells, and
- a region II that can bind to an antibody.

The pharmaceutical composition is typically in the form of a suspension in which NK cells to which the antibody has bound are suspended in a solution, with the binding being stabilized by the substance. The solution for suspending the NK cells is generally, for example, a cryoprotective solution containing DMSO, physiological saline, phosphate buffered saline (PBS), medium, serum, or the like. The solution may contain a pharmaceutically acceptable carrier for pharmaceuticals and quasi-pharmaceuticals.
The production of the pharmaceutical composition of the present invention is preferably performed under conditions that conform to the regulations for manufacturing and quality control of pharmaceuticals and quasi-pharmaceuticals (Good Manufacturing Practice, GMP) and the standards for manufacturing and quality control of products for regenerative medicine etc. (Good Gene, Cellular, and Tissue-based Products Manufacturing Practice, GCTP).
The pharmaceutical composition provided by the present invention can be used for the treatment and/or prevention of various diseases susceptible to highly active NK cells, and so forth. Examples of such diseases are cancers and infectious diseases, and specifically, they include, but not limited to, skin cancer, oral cancer, gallbladder cancer, bile duct cancer, lung cancer, liver cancer, stomach cancer, colon cancer, pancreatic cancer, kidney cancer, ovarian cancer, bladder cancer, prostate cancer, neuroblastoma, leukemia, and infectious diseases caused by viruses, bacteria or the like.
A cell therapy using the pharmaceutical composition of the present invention may be performed solely, or in combination with surgical therapy, chemotherapy, radiation therapy, antibody drug therapy, and so forth.
One of the important findings on which the present invention is based is that NK cells do not injure normal cells at all, with or without binding of antibody drugs, even when they are cultured in vitro for activation. This finding indicates that limiting the effector cells for the ADCC activity of antibody drugs to NK cells improves the safety of the antibody drugs while maintaining the benefit of the ADCC activity. Since NK cells present in the peripheral blood alone are too few in number and the activity thereof is too low to treat malignant tumors and infectious diseases, it is preferable to culture NK cells in vitro for activation. According to the finding mentioned above, effector cells for ADCC activity exerted by antibody drugs can be limited to in vitro-activated NK cells, which makes it easier to use antibody drugs and highly active NK cells in therapies. The finding is particularly important for safety when highly active NK cells are used.

Examples

[Methods commonly used for experiments and examples]

A) Culture method of highly active NK cells GAIA-102

Frozen apheresis blood (HemaCare, PB001CLP) was thawed, and washing and concentration were performed by using Lovo Cell Processing System (Fresenius Kabi) to obtain PBMCs. To the obtained PBMCs, CD3 beads*1 and CD34 beads*2 were added and suspended therein, the suspension was incubated at 4°C for 15 minutes, then a separation buffer*3 was added to the cells to sufficiently suspend them, and the suspension was centrifuged at 300 $\times$ g for 10 minutes. The supernatant was removed, and the cells were suspended in 0.5 mL of the separation buffer so that the maximum cell count should be up to 1 $\times$ $10^8$ cells per one LD column (Miltenyi Biotech, 130-042-901). After 2 mL of the separation buffer was added to an LD column beforehand, the cell suspension was added to the LD column, and the eluate from the LD column was collected. Further, 1 mL of the separation buffer was added to the LD column, and the eluate was collected. The column was then washed with 1 mL of the separation buffer, and the number of the cells in the collected liquids was counted to calculate the total cell count. The cell suspension was centrifuged at 500 $\times$ g for 5 minutes, the supernatant was removed, and then the cells were cultured or frozen at -80°C at a density of 1 \$\times$ $10^7$ cells/mL in STEM-CELLBANKER as CD3- and CD34-depleted PBMCs. When the frozen PBMCs were used for culture, they were thawed by 10-fold dilution with the medium containing serum, and then the culture was started.
The culture was performed by suspending the cells in the KBM501 medium*4 at a density of 1 $\times$ $10^6$ cells/mL using a 6-well plate (Thermo Fisher Scientific, 140675), T-75 flask (Thermo Fisher Scientific, 156499) or adhesion culture bag (Nipro) in a $CO_2$ incubator (37°C, 5% $CO_2$). On day 9 of the culture, the KBM501 medium was added in a volume of 6 mL per well in the case of using the 6-well plate, 50 mL per flask in the case of using the T-75 flask, or 500 mL per

bag in the case of using the bag, and incubation was performed until day 14.

*1: CliniMACS CD3, Miltenyi Biotech, 130-017-601 (5 μL per 1 × $10^7$ cells).
*2: CliniMACS CD34, Miltenyi Biotech, 130-017-501 (2.5 μL per 1 × $10^7$ cells).
*3: PBS (Nacalai Tesque, 14249-24) containing 0.5% human type AB serum (CosmoBio, 12181301, inactivated at 56°C for 30 minutes), and 2 mM EDTA (Thermo Fisher Scientific, 15575-020).
*4: KBM501 (Kohjin Bio, 16025015) containing either 5% human type AB serum (CosmoBio, 12181301, inactivated at 56°C for 30 minutes) or 5% UltraGRO (AventaCell, HPCPLCRL10) supplemented with 2 U/mL of heparin sodium (Nipro).

B) Method for collecting highly active NK cells GAIA-102

On day 14 of the culture, the culture medium was collected, then 1 mM EDTA was added to the culture vessel to separate the adhered cells, and the culture vessel from which the separated cells were collected was washed with the KBM 501 medium. All the collected cell suspensions were centrifuged, and then the cells were washed with and resuspended in the KBM501 medium.

C) Confirmation of binding of GAIA-102 and antibody drug

GAIA-102 cells reacted with each antibody drug was stained with an antibody solution containing each of the following antibodies at a concentration of 1 μg/mL for 30 minutes at 4°C, and then centrifuged (500 × g, 5 minutes), the supernatant was removed, the cells were suspended in PBS, measurement was performed with a flow cytometer (BD LSRFortessa, BD Biosciences), and the results were analyzed with the FlowJo software (FLOWJO, LLC).

<Antibodies used>

Alexa Fluor (registered trademark) 700-labeled anti-human CD56 antibody (Biolegend, 318316), PerCP/C5.5-labeled anti-human CD3 antibody (Biolegend, 300430), PE-Cy7-labeled anti-human CD16 antibody (Biolegend, 302016), and PE-labeled anti-human IgG antibody (SouthemBiotech, 2043-09)

[Experiment 1: Confirmation of injury to Allo-WBC at the time of using antibody drug together]

The number of live NK cells obtained by the procedure described in B) mentioned above was counted, and 1 × $10^7$ cells were suspended in 1 mL of STEM-CELLBANKER, and frozen at -80°C. Then, the cells were thawed on a water bath at 37°C, diluted 10-fold with PlasmaLyte-A, stored at ordinary temperature for 1 hour, then suspended in the KBM501 medium, and cultured for 3 hours.

For the measurement of the cytotoxic activity, a group of thawed NK cells reacted with the K562 cells, a group of K562 cells alone as a negative control, and a group of the K562 cells fixed with 10% formalin as a positive control were prepared.

«NK cells»

The cells cultured for 3 hours after thawing mentioned above were collected and adjusted to a density of 2 × $10^6$ cells/ml in 10% FBS/RPMI 1640.

<<Tumor cell line>>

K562 cells (human chronic myelogenous leukemia cell line) and Hut78 cells (human T lymphoma (Sézary syndrome) cell line) were suspended in serum component-free RPMI 1640 medium, stained by using PKH26 Red Fluorescent Cell Linker Kit (Sigma, PKH26GL-1KT), and finally adjusted to 2 × $10^6$ cells/mL in 10% FBS/RPMI 1640.

«PBMC»

Peripheral blood mononuclear cells (PBMCs) obtained by the procedure of A) mentioned above were used. PBMCs were suspended in serum component-free RPMI 1640 medium, stained with PKH67 Green Fluorescent Cell Linker Kit (Sigma, PKH67GL-1KT), and finally adjusted to 2 × $10^6$ cells/mL in 10% FBS/RPMI 1640.

The NK cells, K562 cells and PBMCs were added to wells of a 96-well plate (IWAKI, 4870-800SP) at a cell

ratio of 1:1 or 1:1:1, Poteligeo was added at a final concentration of 10 μg/ml in the case of Poteligeo(+), and they were mixed and allowed to react at 37°C and 5% $CO_2$ for 2 hours. After the reaction, the mixture was centrifuged (500 × g, 5 min), the supernatant was removed, then a 7-AAD solution diluted with PBS was added to the cells to suspend the cells, and the suspension was incubated at room temperature for 20 minutes. Measurement was performed by using a flow cytometer, and the results were analyzed with the FlowJo software to calculate the cytotoxic activity (% Lysis)*5.

*5: Cytotoxic activity (% Lysis) = (Dead cell rate - Negative control dead cell rate)/(Positive control dead cell rate - Negative control dead cell rate) x 100

The results are shown in Fig. 1. No injury to PBMCs was observed even when Poteligeo was used in combination.

[Experiment 2: Retention of antibody drug by NK cells in culture medium]

The number of viable cells of GAIA-102 obtained by the procedures described in the culture method and collection method for highly active NK cells was counted, and the cells were suspended in PBS (Nacalai Tesque, 14249-24) containing 100 μg/mL of mogamulizumab (Poteligeo Intravenous Infusion 20 mg, Kyowa Kirin) at a density of 1 × $10^6$ cells/mL. The suspended GAIA-102 cells were inoculated in wells of a low-absorption 6-well plate (IWAKI, 4810-800SP) and allowed to stand at room temperature for 1 hour. After the reaction for 1 hour, the cells were collected, and washed three times with PBS. Then, each of GAIA-102 cells suspended in PBS + mogamulizumab, and GAIA-102 cells suspended in the KBM501 medium (5% UltraGRO + 2 U/ml sodium heparin) + mogamulizumab were subjected to antibody staining, and measurement was performed according to the procedures described in C), and the presence or absence of binding of CD16-low or CD16-high GAIA-102 cells in the CD56+ and CD3- cell population and mogamulizumab was analyzed.

The results are shown in Fig. 2. It was found that the GAIA-102 cells cannot retain antibody with CD16 in the KBM501 medium.

[Experiment 3: Effect of additives in culture medium]

The GAIA-102 cells were reacted with 100 μg/mL mogamulizumab at room temperature for 1 hour according to the same procedures as described in Experiment 2, washed 3 times with PBS, and then divided into 5 groups, and the cells of the groups were resuspended in (1) KBM501 medium (5% UltraGRO + 2 U/mL sodium heparin), (2) KBM501 medium not containing serum components (henceforth referred to as serum-free KBM501 medium), (3) 10% FBS/RPMI 1640 medium*5 containing 3,000 IU/mL of IL-2 (Immunace (registered trademark), Shionogi), (4) 10% FBS/RPMI 1640, and (5) PBS. After the cells of each group were allowed to stand for approximately 5 minutes, antibody staining and measurement were performed according to the procedures described in C), and the presence or absence of binding of CD16-low or CD16-high GAIA-102 cells in the CD56+ and CD3- cell population and mogamulizumab was analyzed.
*5: RPMI 1640 medium containing 10% FBS (Nichirei Biosciences, 171012-500ML), 100 units of penicillin, and 100 μg/mL of streptomycin (Nacalai Tesque, 26253-84)

Results are shown in Fig. 3. It was found that the GAIA-102 cells cannot retain antibody with CD 16 in the medium containing UltraGro.

[Experiment 4: Effect in blood]

The GAIA-102 cells were reacted with 100 μg/mL of mogamulizumab at room temperature for 1 hour according to the same procedure as described in Experiment 2, washed three times with PBS, and then divided into 8 groups, and the cells of the groups were resuspended in (1) 10% FBS/KBM501 medium, (2) 5% UltraGRO/KBM501 medium, (3) 1% UltraGRO/KBM501 medium, (4) 0.2% UltraGRO KBM501 medium ((3) and (4) were obtained from 5% UltraGRO/KBM501 medium by 5-fold serial dilution with serum-free KBM501 medium), (5) 5% UltraGRO/RPMI 1640 medium, (6) 5% UltraGRO/RPMI 1640 medium containing 3,000 IU/mL of IL-2, (7) PPP (Platelet-pour Plasma)*6 isolated from peripheral blood collected from healthy volunteers, and (8) peripheral blood (PB) collected from healthy volunteers, respectively. After the cells of each group were allowed to stand for approximately 5 minutes, antibody staining and measurement were performed according to the procedures described in C), and the presence or absence of binding of CD16-low or CD16-high GAIA-102 cells in the CD56+ and CD3- cell population and mogamulizumab was analyzed.
6*: Supernatant obtained by centrifuging peripheral blood at 2,000 × g for 15 minutes at room temperature (brake off).

The results are shown in Fig. 4. Regardless of the type of culture medium, the antibody was removed from the GAIA-102 cells in the presence of UG or blood, indicating that the GAIA-102 cells cannot retain the antibody with CD16

in human blood.

[Experiment 5: Effect of IgG]

The GAIA-102 cells were reacted with 100 μg/mL of mogamulizumab at room temperature for 1 hour by the same procedure as described in Experiment 2, washed three times with PBS, and then divided into 5 groups, and the cells of the groups were resuspended in (1) serum-free KBM501 medium containing 1 mg/mL of trastuzumab (Herceptin (registered trademark) for Injection, Chugai), (2) 5% UltraGRO/KBM501 medium, (3) 5% human type AB serum/KBM501 medium, (4) serum-free KBM501 medium, and (5) PBS, respectively. After the cells of each group were allowed to stand for approximately 5 minutes, antibody staining and measurement were performed according to the procedures described in C), and the presence or absence of binding of CD16-low or CD16-high GAIA-102 cells in the CD56+ and CD3- cell population and mogamulizumab was analyzed.

The results are shown in Fig. 5. It was found that binding of the antibody drug bound to the NK cells was unbound due to competition with other IgGs.

[Example 1: Preparation of prototype substance]

A protein having a region that binds to the antigen NKp46 on the NK cell membrane and a region that binds to IgG was designed, and it was attempted to anchor an antibody drug on the cells. In more detail, the anti-human NKp46 scFv described in WO2017/114694 (SEQ ID NO: 121 in this reference) was used as the region that binds to NKp46. As the region that binds to IgG, the sequence of 373-297aa of the protein G sequence (https://www.uniprot.org/uniprot/P19909) was used.

The preparation of the fusion protein was entrusted to KAICO LTD. (217, Fukuoka City Industry-Academia Collaboration Center, 4-1, Kyudai Shinmachi, Nishiku, Fukuoka-shi, Fukuoka, 819-0388, Japan), and the protein was produced by a recombinant protein expression technique using silkworm.

The amino acid sequence of the designed protein (385 amino acids long) is shown in Fig. 6.

[Example 2: Anchoring of antibody drug on cells]

Using purified prototype substance (pG2-NKp46), an antibody drug was anchored on the GAIA-102 cells, which were obtained by the procedures described as the culture method and collection method for highly active NK cells, by the method described below.

The GAIA-102 cells ($1 \times 10^6$ cells/mL) were inoculated on a low-absorption 96-well plate (IWAKI, 4870-800SP) in a volume of 25 μL per well, and centrifuged (500 × g, 5 minutes), then the supernatant was removed, and the cells were suspended in 25 μL of PBS containing 350 μg/mL of the prototype substance. After the suspension was allowed to stand at room temperature for 1 hour, the cells were washed once with PBS, the suspension medium was replaced with 25 μL of PBS containing 1 μg/mL of Herceptin, and the suspension was allowed to stand at room temperature for another 1 hour. The cells were then washed once with PBS, antibody staining and measurement were performed according to the procedures described in C), and the presence or absence of binding of Herceptin was analyzed.

Herceptin was added at 1 μg/mL to 25 μL of PBS containing 350 μg/mL of the prototype substance in wells of a low-absorption 96-well plate, and the plate was allowed to stand at room temperature for 1 hour. GAIA-102 cells obtained beforehand by inoculating 25 μL of a cell suspension thereof ($1 \times 10^6$ cells/ml), centrifuging them, and removing the supernatant were suspended in the prototype substance + Herceptin solution reacted for 1 hour. The cells were allowed to stand at room temperature for another 1 hour, and washed once with PBS, antibody staining and measurement were performed according to the procedures described in C), and the presence or absence of binding of Herceptin was analyzed.

The GAIA-102 cells were reacted with PBS for 1 hour, subjected to antibody measurement according to the procedure described in C), and used as a control for the two groups mentioned above.

The results are shown in Fig. 7. It was confirmed that anchoring was possible with the prototype substance. It was found that the prototype substance enables anchoring regardless of the order of mixing with the antibody or GAIA-102 cells.

[Example 3: Effect on various antibody drugs]

GAIA-102 cells ($1 \times 10^5$ cells) obtained by the procedures described in the culture method and collection method for highly active NK cells were suspended in 100 μL of 5% UltraGRO/KBM501 medium containing 13.3 μg/mL of the prototype substance, and allowed to stand at room temperature for 1 hour. After 1 hour, the cells were washed once with PBS, and divided into 5 groups. The cells of the groups were suspended in PBS containing 1 μg/mL of one

of various antibody drugs, cetuximab (Erbitux (registered trademark) Injection, Merck Biopharma), Herceptin, Poteligeo, rituximab (Rituxan (registered trademark) Intravenous Infusion, Zenyaku Kogyo), and dinutuximab (Unituxin, United Therapeutics), respectively. As controls, the GAIA-102 cells were similarly suspended in PBS containing each antibody drug (without anchoring by the prototype substance). The cell suspensions were allowed to stand at room temperature for 1 hour, the cells were washed once with PBS, antibody staining and measurement were performed according to the procedures described in C), and the presence or absence of binding of each antibody drug was analyzed.

The results are shown in Fig. 8. It was found that binding between the cells and antibody was stabilized by anchoring with the prototypic substance for all the antibody drugs.

Sequence Listing Free Text

SEQ ID NO: 1, Nucleotide sequence, pG/Fc-NKp46/scFv
SEQ ID NO: 2, Amino acid sequence, pG/Fc-NKp46/scFv
SEQ ID NOS: 3 to 12, and 32 to 43, Domains capable of binding to NK cell surface protein
SEQ ID NOS: 13 to 31, sequences of SEQ ID NOS: 1 to 17, 36 and 37 described in WO2016/186206

## Claims

**1.** A method for stabilizing binding of an antibody and an NK cell, which uses a substance having:

- a region I that can bind to a surface protein of the NK cell, and
- a region II that can bind to the antibody.

**2.** The method according to claim 1, wherein the surface protein of the NK cell is one selected from the group consisting of NKp46, NKp30, NKG2D, IL-15R, IL-2R, KIR3DS1, NKG2C, NKp80, NKp65, NKp44, LALRA1, LILRA2, DNAM-1, and 2B4.

**3.** The method according to claim 1 or 2, wherein at least one of the region I and the region II is a single chain Fv fragment (scFv).

**4.** The method according to any one of claims 1 to 3, wherein the antibody is an antibody drug for a treatment of a cancer or infectious disease.

**5.** A method for producing a population of NK cells, which comprises the steps of:

(1) preparing a population of NK cells, an antibody, and a substance having a region I that can bind to a surface protein of the NK cells and a region II that can bind to the antibody; and
(2) adding the antibody to the population of NK cells in the presence of the substance to obtain a population of NK cells bound to the antibody, so that the NK cells are bound to the region I of the substance, the antibody is bound to the region II of the substance, and the substance stabilizes the binding between the antibody and the NK cells.

**6.** The production method according to claim 5, wherein the antibody is an antibody drug for a treatment of a cancer or infectious disease.

**7.** A pharmaceutical composition comprising a population of NK cells to which an antibody drug is bound, wherein binding of the antibody drug and the NK cells is stabilized by a substance having the following regions:

- a region I that can bind to a surface protein of the NK cells, and
- a region II that can bind to the antibody.

[Fig.1]

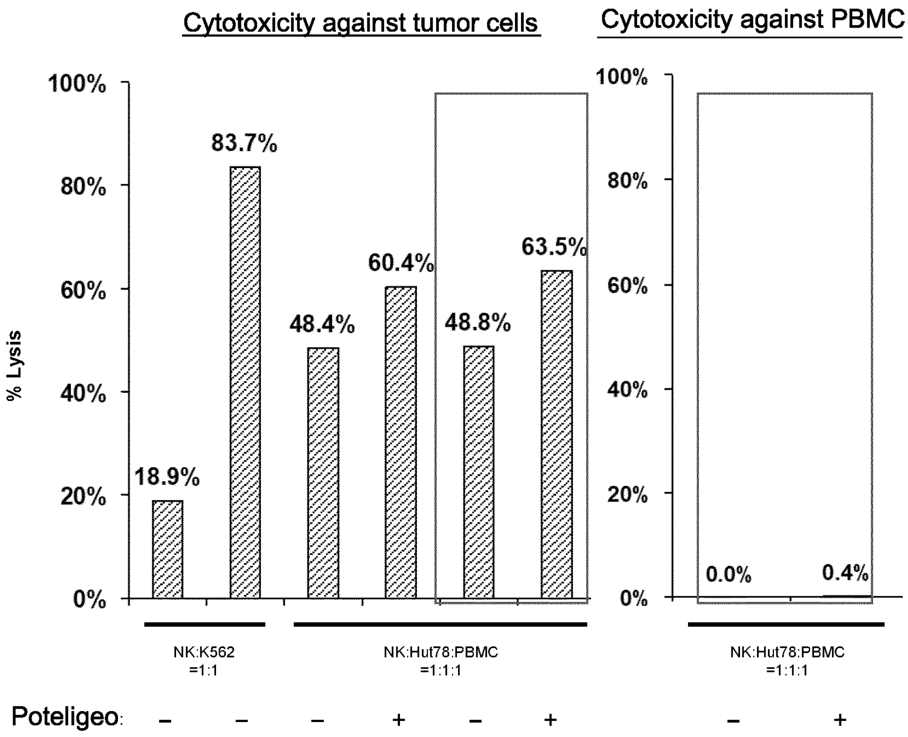
Cytotoxicity against tumor cells
Cytotoxicity against PBMC
% Lysis
100%
80%
60%
40%
20%
0%
18.9%
83.7%
48.4%
60.4%
48.8%
63.5%
0.0%
0.4%
NK:K562
=1:1
NK:Hut78:PBMC
=1:1:1
NK:Hut78:PBMC
=1:1:1
Poteligeo:
–
–
–
+
–
+
–
+

[Fig.2]

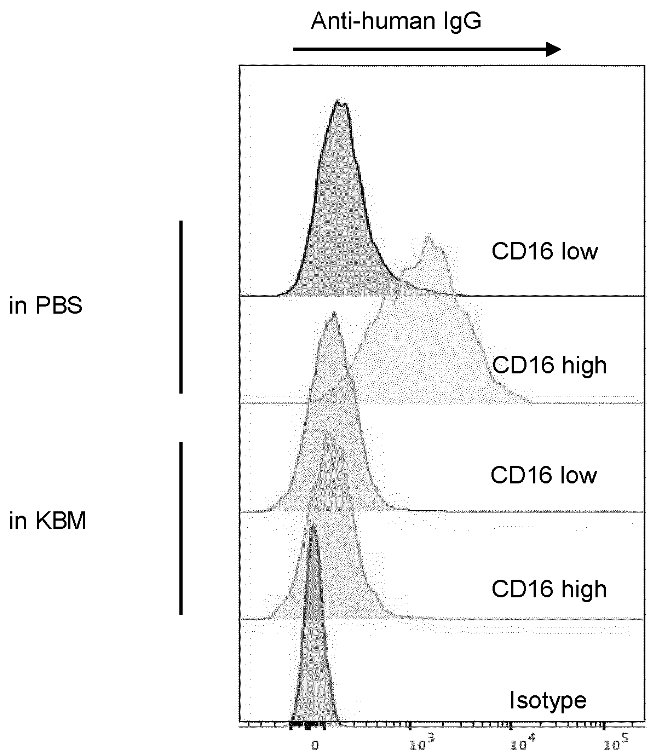
Anti-human IgG
in PBS
in KBM
CD16 low
CD16 high
CD16 low
CD16 high
Isotype
0
$10^3$
$10^4$
$10^5$

[FIG.3]

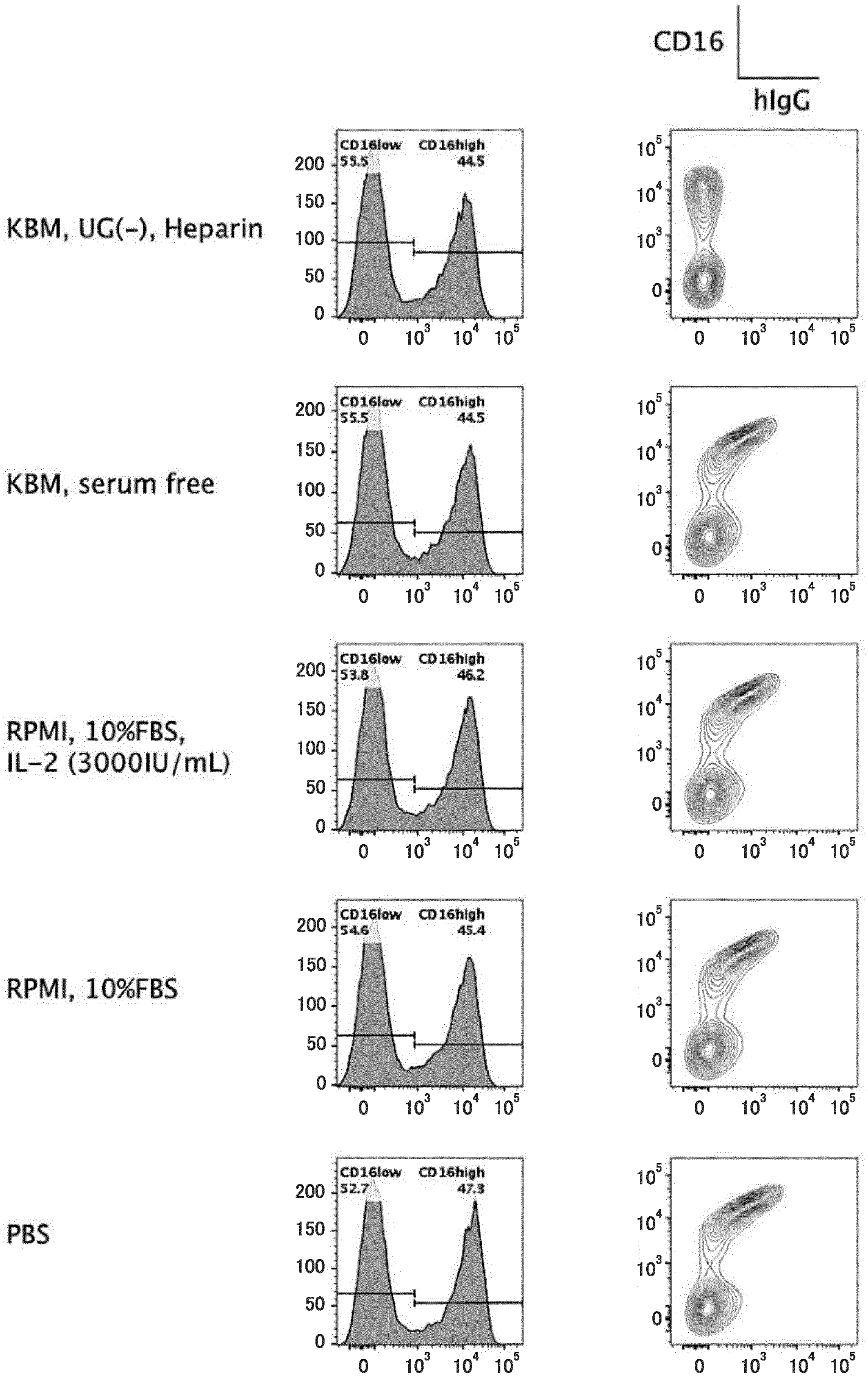
CD16
hIgG
KBM, UG(-), Heparin
CD16low 55.5
CD16high 44.5
KBM, serum free
CD16low 55.5
CD16high 44.5
RPMI, 10%FBS, IL-2 (3000IU/mL)
CD16low 53.8
CD16high 46.2
RPMI, 10%FBS
CD16low 54.6
CD16high 45.4
PBS
CD16low 52.7
CD16high 47.3
200
150
100
50
0
0
$10^3$
$10^4$
$10^5$

[Fig.4]

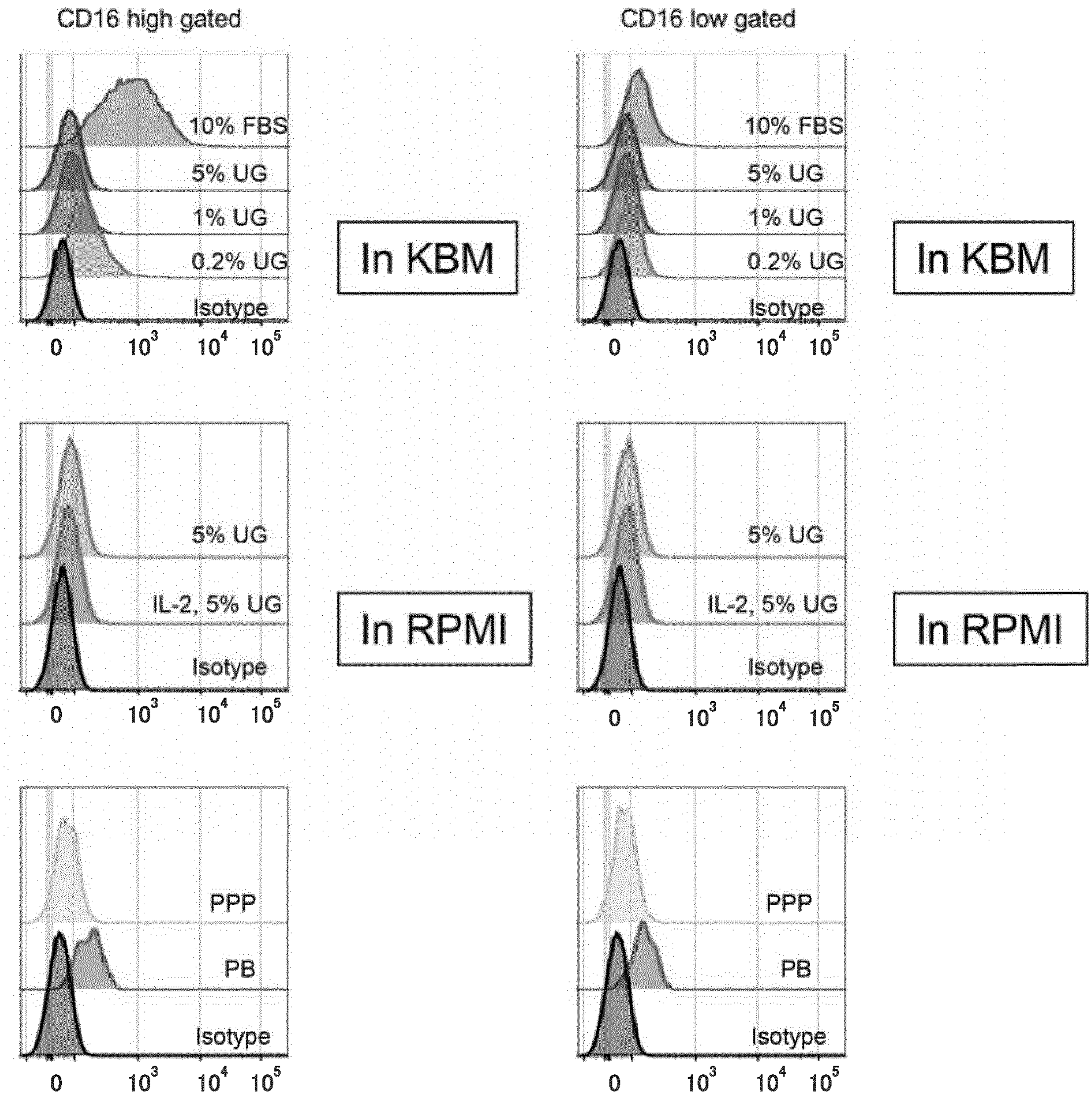
CD16 high gated
CD16 low gated
10% FBS
5% UG
1% UG
0.2% UG
Isotype
In KBM
IL-2, 5% UG
In RPMI
PPP
PB
0
$10^3$
$10^4$
$10^5$

[Fig.5]

CD16 high gated：

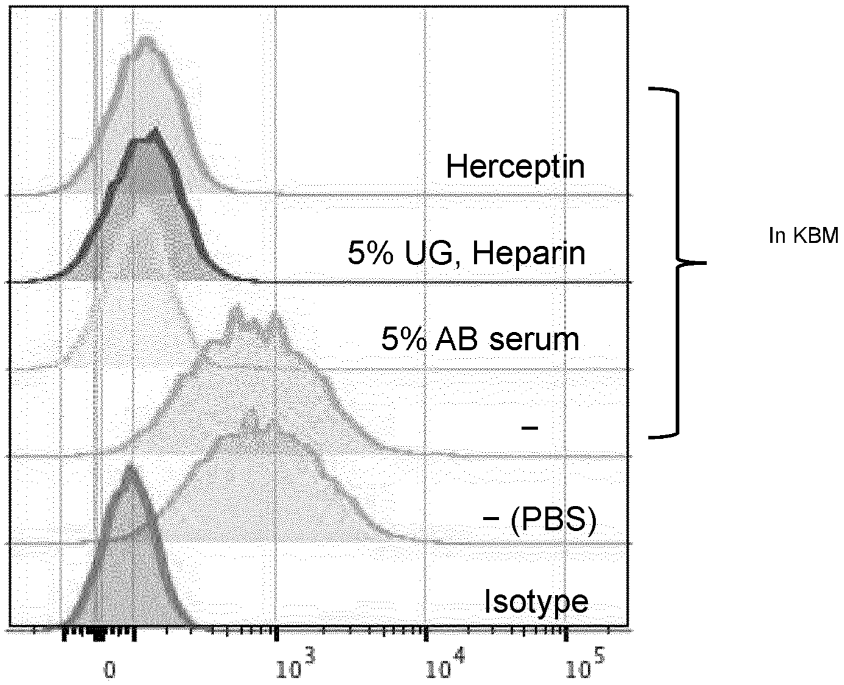


CD16 low gated：

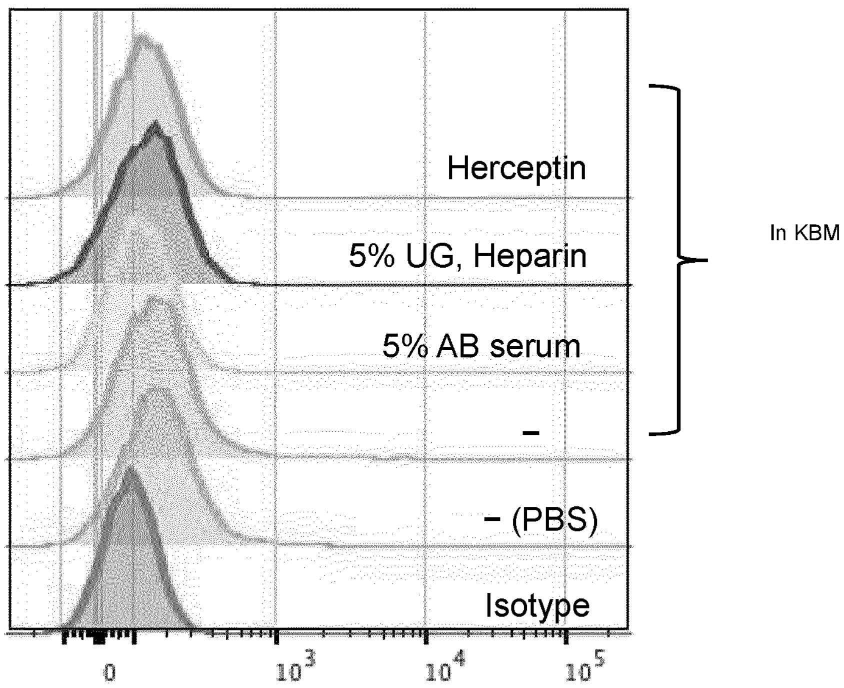

[Fig.6]

**Amino acid sequence of the designed prototype substance** (SEQ ID NO:2, (20)..(404))

TYKLVINGKTLKGETTTEAVDAATAEKVFKQYANDNGVDGEWTYDDATKTFTVTEKPEVIDASEL
TPAVTTYKLVINGKTLKGETTTKAVDAETAEKAFKQYANDNGVDGVWTYDDATKTFTVTEGGGG
SGGGGSGGGGSstgsevqlqqsgpelvkpgasvkiscktsgytfteytmhwvkqshgkslewiggispniggtsynqkfkgk
atltvdksssstaymelrsltsedsavyycarrggsfdywgqgttltvssveggsggsggsggsggvddivmtqspatlsvtpgdrvslscr
asqsisdylhwyqqkshesprllikyasqsisgipsrfsgsgsgsdftlsinsvepedvgvyycqnghsfpltfgagtklelk

**Nucleotide sequence containing a part encoding the designed prototype substance** (SEQ ID NO:1)

ATGCGGCTCACACTATTCGCTTTCGTACTCGCTGTGTGTGCCCTCGCAAGCAACGCTACGTA
CAAATTAGTTATAAACGGTAAAACGCTGAAAGGTGAGACCACTACAG AAGCCGTTGACGCT
GCTACGGCGGAAAAGGTATTCAAACAGTATGCAAACGATAACGGCGTTGACGGTGAGTGGA
CCTACGACGATGCGACAAAAACCTTTACAGTTA CTGAAAAGCCTGAGGTCATCGATGCCTC
GGAGCTGACACCGGCGGTGACAACGTATAAGTTGGTCATAAATGGGAAGACACTGAAGGG
CGAGACCACCACTAAAGCT GTGGACGCGGAGACGGCCGAAAAGGCATTCAAACAATACGC
TAATGATAATGGAGTTGATGGTGTGTGGACGTATGACGACGCAACGAAAACTTTTACTGTCA
CGGAAGGGGGTGGGGGGTCAGGAGGTGGAGGCAGTGGGGGAGGGGGTTCGtccacaggatct
gaagtccaattgcagcaatcgggccctgagctcgtcaagccaggcgc aagtgttaaaatttcgtgtaagactagcggttatacctttacc
gagtatacaatgcattgggtaaagcaaagccatggcaagtccctggaatggatcggaggaatatctc cgaacatcggtggcaccagcta
taatcaaaagtttaaagggaaggcgaccctaacggtggacaagagttcttctaccgcctacatggagctccgcagcctaacgagtg aa
gattctgccgtgtactattgtgcgagacgtggtgggtcctttgactattgggggcagggtacaacactgactgtttcgtctgtcgaaggtggtt
ctggcggcagt gggggctcggggggttcaggtggggtggacgacattgtgatgacccagtctcctgcgacattaagtgtaacgccgg
gggatcgtgtgtccctgtcttgtcgtgcct cacaatctattagtgattacctgcactggtaccaacagaagtcccatgagagtccacgcctatt
aatcaaatacgcctcgcagagcatctcaggcattccgtccaggttc agcgggtccggaagtgggtcggactttacattaagcattaactca
gttgaaccagaggacgtaggagtttactactgccaaaatggccattcattccccctaactttcg gtgcgggtacgaaactagaattgaaa
GAGAATCTTTATTTCCAGGGCCACCATCACCACCATCACTGGTCTCATCCACAATTTGAGAAG
CACCATCATCATCACCAC

[Fig.7]

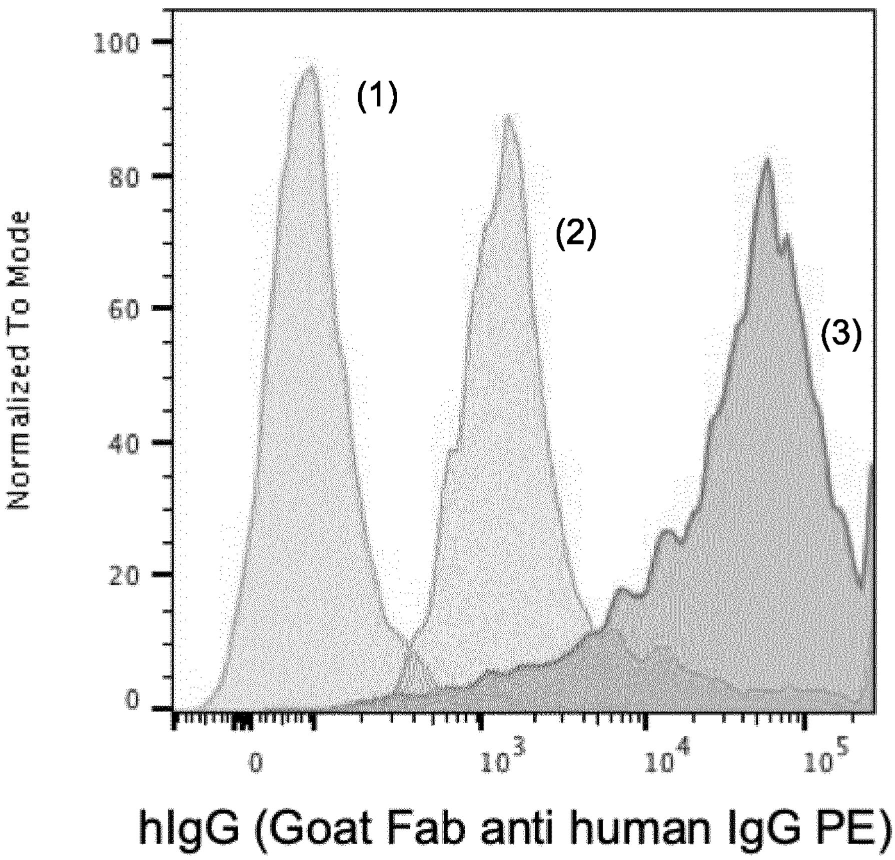
100
80
60
40
20
0
Normalized To Mode
(1)
(2)
(3)
0
10^3
10^4
10^5
hIgG (Goat Fab anti human IgG PE)

[Fig.8]

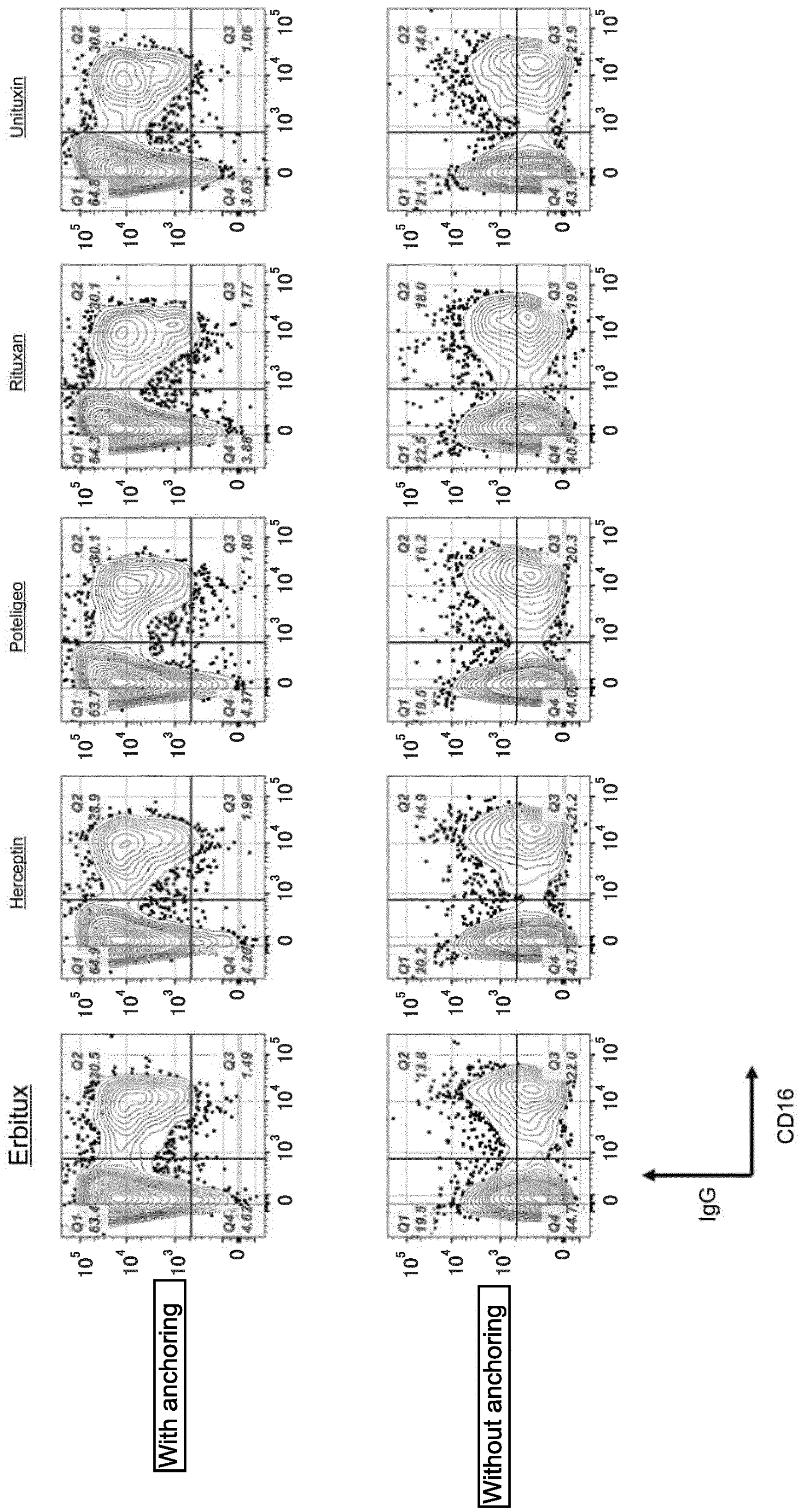
Erbitux
Herceptin
Poteligeo
Rituxan
Unituxin
With anchoring
Without anchoring
Q1
Q2
Q3
Q4
63.4
30.5
1.49
4.62
64.9
28.9
1.98
4.20
63.7
30.1
1.80
4.37
64.3
30.1
1.77
3.86
64.8
30.6
1.06
3.53
19.5
13.8
22.0
44.7
20.2
14.9
21.2
43.7
19.5
16.2
20.3
44.0
22.5
18.0
19.0
40.5
21.1
14.0
21.9
43.1
IgG
CD16

# INTERNATIONAL SEARCH REPORT

International application No.
PCT/JP2021/024808

A. CLASSIFICATION OF SUBJECT MATTER
A61K 47/42(2017.01)i; A61K 35/17(2015.01)i; A61K 39/395(2006.01)i; A61P 31/00(2006.01)i; A61P 35/00(2006.01)i; C07K 16/18(2006.01)i; C07K 19/00(2006.01)i; C12N 5/0783(2010.01)i
FI: A61K47/42; A61K35/17 A ZNA; A61K39/395 D; A61K39/395 T; A61P31/00; A61P35/00; C07K16/18; C07K19/00; C12N5/0783
According to International Patent Classification (IPC) or to both national classification and IPC

B. FIELDS SEARCHED

Minimum documentation searched (classification system followed by classification symbols)
A61K47/42; A61K35/17; A61K39/395; A61P31/00; A61P35/00; C07K16/18; C07K19/00; C12N5/0783

Documentation searched other than minimum documentation to the extent that such documents are included in the fields searched

| | |
|---|---|
| Published examined utility model applications of Japan | 1922-1996 |
| Published unexamined utility model applications of Japan | 1971-2021 |
| Registered utility model specifications of Japan | 1996-2021 |
| Published registered utility model applications of Japan | 1994-2021 |

Electronic data base consulted during the international search (name of data base and, where practicable, search terms used)
JSTPlus/JMEDPlus/JST7580 (JDreamIII); CAplus/MEDLINE/EMBASE/BIOSIS (STN)

C. DOCUMENTS CONSIDERED TO BE RELEVANT

| Category* | Citation of document, with indication, where appropriate, of the relevant passages | Relevant to claim No. |
|---|---|---|
| Y | JP 2019-500032 A (UCB BIOPHARMA SPRL) 10 January 2019 (2019-01-10) in particular, claims, paragraphs [0058], [0064], [0079], [0084], example 4, fig. 3, 5 | 1-7 |
| Y | JP 2018-502068 A (1GLOBE BIOMEDICAL CO., LTD.) 25 January 2018 (2018-01-25) in particular, claims, paragraphs [0013], [0062]-[0063] | 1-7 |
| Y | JP 2019-503713 A (INNATE PHARMA S.A.) 14 February 2019 (2019-02-14) in particular, claims, paragraphs [0030], [0033] | 1-7 |

☒ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 19 August 2021 (19.08.2021) | 31 August 2021 (31.08.2021) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No.
PCT/JP2021/024808

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2018-516881 A (UNIVERSITY HEALTH NETWORK) 28 June 2018 (2018-06-28) in particular, claims, paragraph [0033] | 1-7 |
| Y | WO 2018/207900 A1 (GAIA BIOMEDICINE INC.) 15 November 2018 (2018-11-15) in particular, claims | 1-7 |

Form PCT/ISA/210 (continuation of second sheet) (January 2015)

**INTERNATIONAL SEARCH REPORT**
Information on patent family members

International application No.
PCT/JP2021/024808

| Patent Documents referred in the Report | Publication Date | Patent Family | Publication Date |
|---|---|---|---|
| JP 2019-500032 A | 10 Jan. 2019 | US 2018/0355063 A1<br>in particular, claims, paragraphs [0132], [0140], [0177], [0182], example 4, fig. 3, 5<br>WO 2017/093410 A1<br>EP 3383901 A1<br>CN 108473557 A | |
| JP 2018-502068 A | 25 Jan. 2018 | US 2017/0362299 A1<br>in particular, claims, paragraphs [0013], [0063]-[0064]<br>WO 2016/094456 A1<br>EP 3230311 A1<br>CN 107108718 A<br>KR 10-2017-0089003 A | |
| JP 2019-503713 A | 14 Feb. 2019 | US 2018/0355036 A1<br>in particular, claims, paragraphs [0039], [0042]<br>WO 2017/114694 A1<br>EP 3397645 A1<br>CN 108779175 A | |
| JP 2018-516881 A | 28 Jun. 2018 | US 2018/0153938 A1<br>in particular, claims, paragraph [0032]<br>WO 2016/176756 A1<br>EP 3291833 A1 | |
| WO 2018/207900 A1 | 15 Nov. 2018 | EP 3623467 A1<br>in particular, claims<br>CN 110603320 A<br>JP 2018-193303 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2018193303 A
- JP 2009500346 A
- WO 2017114694 A
- JP 2019503713 A
- JP 2020506971 A
- WO 2001045746 A
- JP 2016088906 A
- US 7408030 B
- WO 2013027796 A
- WO 2011148952 A
- WO 2016186206 A
- WO 2007004748 A
- JP 2019170176 A
- JP 2020035297 A


### Non-patent literature cited in the description


- **UCHIDA J. et al.** *J. Exp. Med.,* 2004
- **SUGATA K. et al.** *Sci. Rep.,* 2016
- *Science,* 2000, vol. 287, 1279-1283
- *Chem. Bio. Chem.,* 2005, vol. 6, 1242-1253
- *J. Chromatography,* 1992, vol. 604, 29-37
- *J. Molecular Recognition,* 1998, vol. 11, 128-133
- *J. Immunological Methods,* 2002, vol. 271, 77-88
- *J. Biol. Chem.,* 2009, vol. 284, 9986-9993
- *J. Biol. Chem.,* 2012, vol. 287, 43126-43136
- *Materials,* 2016, vol. 9, 994
- *RNA,* 2008, vol. 14, 1154-1163
- *J. Biomedical Materials Res. A,* 2003, vol. 65, 379-385
- *J. Appl. Polym. Sci.,* vol. 89, 1567-1572
- *J. Chromatography B,* 2003, vol. 795, 93-103
- *J. Chromatography B,* 2000, vol. 740, 1-15
- *J. Chromatography B,* 2003, vol. 792, 177-185
- **LEONG JW et al.** *Biol. Blood Marrow Transplant,* 2014, vol. 20, 463-473
- *Nature Biotechnology,* 2005, vol. 23
- *Front Immunol.,* June 2017, vol. 9 (8), 653